# EUROPÄISCHE PATENTSCHRIFT

(11) **EP 3 031 816 B1**
(45) Veröffentlichungstag und Bekanntmachung des Hinweises auf die Patenterteilung: **27.02.2019**
(21) Anmeldenummer: 15185814.9
(22) Anmeldetag: 04.12.2014
(51) Int. Cl.: C07F 9/6574, C07B 41/06, C07C 45/50, C07F 9/145

(54) **TERPHENYL-2-OXY-PHOSPHITE ALS LIGANDEN IN HYDROFORMYLIERUNGS-KATALYSATOREN**
TERPHENYL-2-OXY-PHOSPHITE AS LIGANDS IN HYDROFORMYLATION CATALYSTS
TERPHENYL-2-OXY-PHOSPHITES EN TANT QUE LIGAND DANS CATALYSATEURS DE LA HYDROFORMYLATION

(43) Veröffentlichungstag der Anmeldung: 15.06.2016
(62) Teilanmeldung aus: 14196185.4
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Fridag, Dirk, 45721 Haltern am See (DE); Hess, Dieter, 45770 Marl (DE); Geilen, Frank, 45721 Haltern am See (DE); Börner, Armin, 18059 Rostock (DE); Selent, Detlef, 18059 Rostock (DE); Uhlemann, Marcus, 65835 Liederbach am Taunus (DE)

(56) Entgegenhaltungen:
- EP-A1- 2 567 949
- WO-A1-85/03702

## Beschreibung

Die Erfindung betrifft Monophosphite, die ein Diphenylphenol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor PIII. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

In EP 0 155 508 B1 werden unterschiedliche Monophosphite aufgezeigt. Die abgebildeten Monophosphite weisen alle eine symmetrische Biphenyleinheit auf, d.h. die beiden Phenylringe lassen sich durch eine zwischen ihnen liegende Spiegelebene aufeinander abbilden. Die einzige Ausnahme stellt die Verbindung 16 in Tabelle 6 (Seite 58) dar. Dieser Ligand wurde nur zu Vergleichsversuchen verwendet, und weist mit einem Wert von 0,22 eine niedrige Umsatzrate auf.

In WO 85/03702 werden Diorganophosphite beschrieben. Diese werden als Liganden für einen Komplex verwendet, mit welchem Hydroformylierungsreaktionen katalysiert werden.

In EP 2 567 949 A1 wird ein Verfahren zur Abtrennung von 1-Buten aus C₄-haltigen Kohlenwasserstoffgemischen beschrieben. Die Gemische enthalten des weiteren Isobuten. Die Abtrennung erfolgt hierbei durch Hydroformylierung unter Einsatz von Bisphosphitliganden.

Der Erfindung lag die Aufgabe zugrunde, Monophosphite bereitzustellen, welche gegenüber den bekannten Monophosphiten vorteilhafte Eigenschaften in der Hydroformylierung aufweisen. Insbesondere bestand die Aufgabe darin, neue Liganden bereitzustellen, deren Einsatz gegenüber strukturverwandten Monophosphiten, welche ebenfalls eine Biphenoleinheiten aufweisen, zu einer verbesserten Ausbeute führen. Die verbesserte Ausbeute sollte bei zumindest einem Olefin realisiert werden.

Die Aufgabe wird gelöst durch eine Verbindung nach Anspruch 1.

Verbindung, welche die allgemeine Struktur **III** aufweist: 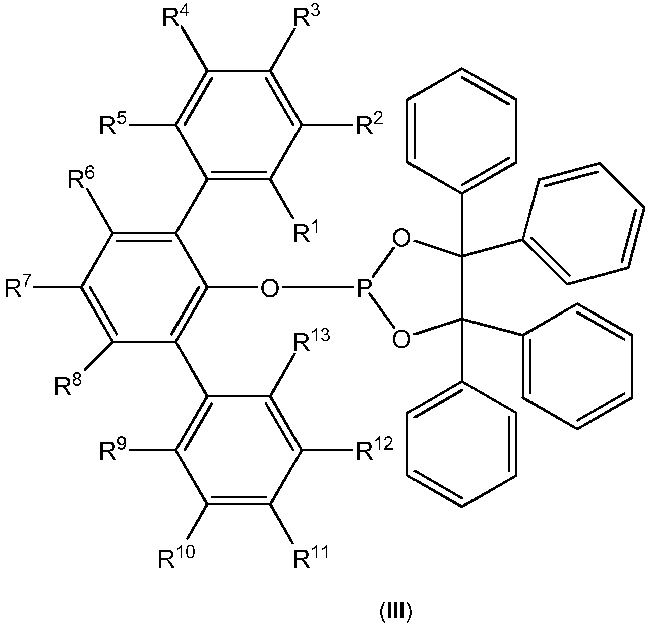 wobei
R¹, R², R³, R⁴, R⁵, R⁶, R⁷, R⁸, R⁹, R¹⁰, R¹¹, R¹², R¹³ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
wobei die genannten Alkyl- und Arylgruppen wie folgt substituiert sein können:
   substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
   substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.

In einer Ausführungsform sind R¹, R², R³, R⁴, R⁵, R⁶, R⁷, R⁸, R⁹, R¹⁰, R¹¹, R¹², R¹³ ausgewählt aus:
-H, -(C₁-C₁₂)-Alkyl, -O-(C₁-C₁₂)-Alkyl, -O-(C₆-C₂₀)-Aryl.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.
Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Synthese

### Biphenole

Die Synthese der Biphenole erfolgt analog zu DE102013203865 und DE102013203867.

### 2,6-Diphenylphenoxydichlorphosphin

### (Vorstufe)

Chlorophosphite lassen sich durch Zusatz von Phosphortrichlorit in Gegenwart einer Base herstellen. Die Synthese des 2,6-Diphenylphenoxydichlorphosphin erfolgte gemäß "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### 6-([1,1':3',1"-Terphenyl]-2'-yloxy)dibenzo[d,f][1,3,2]dioxaphosphepin.

### (Vergleichsverbindung)

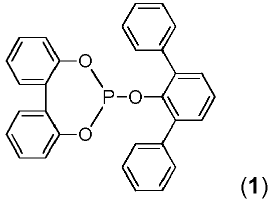

Eine Lösung von 2,6-Diphenylphenol (0,491 g; 1,99 mmol) in Toluol (9 ml) wurde mit Triethylamin (1,851 g; 18,293 mmol) versetzt und die erhaltene Mischung tropfenweise bei 0°C zu einer Lösung von 6-Chlorodibenzo[*d*,*f*][1,3,2]dioxaphosphepin (0,500 g; 1,99 mmol) in Toluol (9 ml) gegeben. Die Reaktionsmischung wurde über Nacht gerührt und dann filtriert. Das Filtrat wurde im Vakuum bis zur Trockne eingeengt und der erhaltene Rückstand aus heißem Toluol (3 ml) umkristallisiert. Ausbeute: 0,536 g (1,164 mmol; 58 %). Elementaranalyse (berechnet für C₃₀H₂₁O₃P = 460,44 g/ mol) C 78,30 (78,25); H 4,72 (4,60) %.
³¹P-NMR (CD₂Cl₂): 146,0 ppm.
¹H-NMR (CD₂Cl₂): 6,12 (m, 2 H); 7,21 (m, 4 H); 7,35-7,40 (m, 3 H); 7,43-7,46 (m, 2 H); 7,52-7,57 (m, 6 H); 7,59-7,64 (m, 4 H) ppm.
¹³C-NMR (CD₂Cl₂): 122,5; 125,2; 125,5; 127,9; 128,9; 129,5; 129,8; 131,1; 131,2; 136,4; 136,5; 138,9; 146,5 (d, *J*_{CP}= 8,5 Hz); 149,0 (d, *J*_{CP}= 4,7 Hz) ppm.
ESI-TOF/HRMS: m/e 461,13018 (M+H)⁺.

### 6-([1,1':3',1"-Terpheny)]-2'-y)oxy)-4,8-di-tert-buty)-2,10-dimethoxydibenzo[d,f][1,3,2]dioxaphosphepin.

### (Vergleichsverbindung)

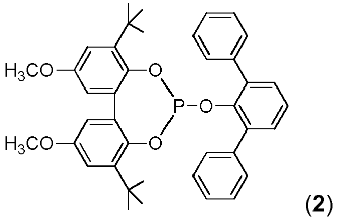

Eine Lösung von 2,6-Diphenylphenol (0,411 g; 1,65 mmol) in Toluol (8 ml) wurde mit Triethylamin (1,529 g; 15,11 mmol) versetzt und die erhaltene Mischung tropfenweise bei 0°C zu einer Lösung von 4,8-Di-*tert*-butyl-6-chloro-2,10-dimethoxydibenzo[*d*,*f*][1,3,2]dioxaphosphepin (0,697 g; 1,65 mmol) in Toluol (6 ml) gegeben. Die Reaktionsmischung wurde über Nacht bei Raumtemperatur und für 5 h bei 70 °C gerührt. Dann wurde die Mischung filtriert und das Filtrat im Vakuum bis zur Trockne eingeengt. Der erhaltene Rückstand wurde aus Hexan (4 ml) umkristallisiert. Ausbeute: 0,417 g (0,659 mmol; 40 %).

Elementaranalyse (berechnet für C₄₀H₄₁O₅P = 632,70 g/ mol) C 75,95 (75,93); H 6,52 (6,53); P 5,01 (5,00) %.
³¹P-NMR (CD₂Cl₂): 140,9 ppm.
¹H-NMR (CD₂Cl₂): 1,37 (s, 18 H); 3,84 (s, 6 H); 6,51 (d, ⁵*J*_{HH}= 3,1 Hz, 2 H, Hₐᵣₒₘ); 6,89-7,95 (m, br, 15 H, Hₐᵣₒₘ) ppm.
¹³C-NMR (CD₂Cl₂): 31,1; 35,5; 55,9; 113,0; 114,5; 125,2; 126,5-131,0 (broad, overlapping signals); 133,8 (d, *J*_{CP}= 4,0 Hz); 141,5 (d, *J*_{CP}= 6,3 Hz); 142,6; 144,8; 156,1 ppm.
ESI-TOF/HRMS: m/e 633,27654 (M+H)⁺.

### 6-([1,1':3',1"-Terpheny)]-2'-yloxy)-4-methoxy-2-methylbenzo[d]naphtho[1,2-f][1,3,2]dioxaphosphepin.

### (Vergleichsverbindung)

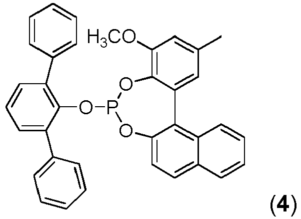

Eine Lösung von 1-(2-Hydroxy-3-methoxy-5-methylphenyl)naphthalin-2-ol (0,468 g; 1,670 mmol) in Toluol (12 ml) wurde mit Triethylamin (1,548 g; 15,296 mmol) versetzt und auf 0 °C gekühlt. Zu dieser Mischung wurde eine Lösung von 2,6-Diphenylphenoxydichlorphosphin (0,580 g; 1,670 mmol) in Toluol (3 ml) tropfenweise zugegeben. Die Reaktionsmischung wurde über Nacht bei Raumtemperatur und für 2 h bei 70 °C gerührt. Die Mischung wurde über Kieselgel filtriert und das Filtrat im Vakuum bis zur Trockne eingeengt. Der erhaltene Feststoff wurde bei 50°C / 0,1 mbar getrocknet und durch Säulenchromatografie gereinigt (Hexan/Dichlormethan, 1:2, R*_{f}* = 0,5). Ausbeute: 0,760 g (1,370 mmol; 97 %). Elementaranalyse (berechnet für C₃₆H₂₇O₄P = 554,58 g/ mol) C 78,04 (77,97); H 4,94 (4,91); P 5,53 (5,58) %.
³¹P-NMR (CD₂Cl₂): 145,5; 149,4 ppm.
¹H-NMR (CD₂Cl₂): 2,39-2,54 (2s, 3 H); 3,77-3,95 (2s, 3 H); 6,16 (m, 1 H, Hₐᵣₒₘ); 6,79 (m, 1 H, Hₐᵣₒₘ); 6,91-6,94 (m, 1 H, Hₐᵣₒₘ); 7,29-7,40 (m, 2 H, Hₐᵣₒₘ); 7,40-7,46 (m, 7 H, Hₐᵣₒₘ); 7,50-7,55 (m, 2 H, Hₐᵣₒₘ); 7,55-7,65 (m, 5 H, Hₐᵣₒₘ); 7,90-7,95 (m, 1 H, Hₐᵣₒₘ); 8,05-8,11 (m, 1 H, Harom) ppm.
¹³C-NMR (CD₂Cl₂): 21,6; 56,1; 112,9; 121,8; 124,3; 125,2; 126,0; 126,8; 127,7; 128,4; 128,6; 128,8; 129,5; 130,6; 131,3; 131,8; 132,1; 135,0; 135,6; 136,5 (d, *J*_{CP}= 4,3 Hz); 138,9; 146,3 (d, *J*_{CP}= 6,8 Hz); 147,3 (d, *J*_{CP}= 6,6 Hz); 152,4 (d, *J*_{CP}= 2,5 Hz) ppm.
ESI-TOF/HRMS: m/e 555,17222 (M+H)⁺.

### 6-([1,1':3',1"-Terphenyl]-2'-yloxy)-4-methoxy-2,10-dimethyldibenzo[d,f][1,3,2]dioxaphosphepin.

### (Vergleichsverbindung)

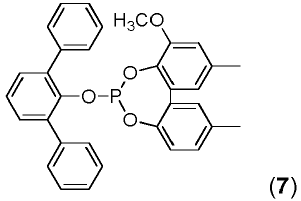

Eine gerührte Lösung von 3-Methoxy-5,5'-dimethyl-[1,1'-biphenyl]-2,2'-diol (0,465 g; 1,903 mmol) in Toluol (12 ml) wurde mit Triethylamin (1,764 g; 17,433 mmol) versetzt und auf 0 °C gekühlt. Zu dieser Mischung wurde eine Lösung von 2,6-Diphenylphenoxydichlorphosphin (0,661 g; 1,903 mmol) in Toluol (3 ml) tropfenweise zugegeben. Die Reaktionsmischung wurde über Nacht gerührt und dann filtriert. Das Filtrat wurde im Vakuum bis zur Trockne eingeengt und der erhaltene Feststoff aus Hexan (20 ml) umkristallisiert. Ausbeute: 0,804 g (1,550 mmol; 82 %).

Elementaranalyse (berechnet für C₃₃H₂₇O₄P = 518,55 g/ mol) C 76,38 (76,44); H 5,18 (5,25); P 5,97 (5,97) %.
³¹P-NMR (CD₂Cl₂): 144,9 ppm.
¹H-NMR (CD₂Cl₂): (m, 6 H); 3,75 (s, 3 H); 5,86 (m, 1 H, Hₐᵣₒₘ); 6,76 (m, 2 H, Hₐᵣₒₘ); 6,92 (m, 1 H, Hₐᵣₒₘ)) 7,19 (m, 1 H, Hₐᵣₒₘ); 7,36 (m, 1 H, Hₐᵣₒₘ); 7,40-7,55 (m, 8 H, Hₐᵣₒₘ)) 7,60-7,68 (m, 4 H, Hₐᵣₒₘ) ppm.
¹³C-NMR (CD₂Cl₂): 20,9; 21,6; 56,0; 112,9; 121,4; 121,8; 125,1; 127,7; 128,8; 129,9; 130,1; 130,7; 131,3; 132,7 (d, *J*_{CP}= 3,5 Hz); 134,4; 135,1; 135,7; 136,4 (d, *J*_{CP}= 4,4 Hz); 139,0; 146,4 (d, *J*_{CP}= 6,8 Hz); 147,6 (d, *J*_{CP}= 7,4Hz); 152,0 (d, *J*_{CP}=2,5 Hz) ppm.
ESI-TOF/HRMS: m/e 519.17235 (M+H)⁺.

### 2-([1,1':3',1"-Terphenyl]-2'-yloxy)-4,4,5,5-tetraphenyl-1,3,2-dioxaphospholan.

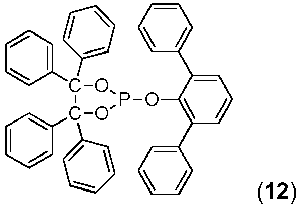

Eine Lösung von 2,6-Diphenylphenol (0,614 g; 2,493 mmol) in THF (5 ml) wurde mit einem Äquivalent n-Butyllithium, gelöst in Hexan (8 ml), bei -20 °C versetzt. Die Mischung wurde für 20 min bei -20°C gerührt und auf Raumtemperatur erwärmt. Dann wurde eine Lösung von 2-Chloro-4,4,5,5-tetraphenyl-1,3,2-dioxaphospholan (1,074 g; 2,493 mmol) in THF (6 ml) tropfenweise zugegeben. Die Reaktionsmischung wurde über Nacht bei Raumtemperatur gerührt. Das Lösungsmittel wurde im Vakuum abgezogen, dann Toluol (15 ml) zugegeben und die resultierende Lösung zweimal filtriert. Das Filtrat wurde bis zur Trockne eingedampft. Der erhaltene hellgelbe Feststoff wurde für 4 h bei 50 °C getrocknet. Ausbeute: 1,448 g (2,260 mmol; 91 %).

Elementaranalyse (berechnet für C₄₄H₃₃O₃P = 640,72 g/ mol) C 82,32 (82,48); H 5,39 (5,19); P 4,94 (4,84) %.
³¹P-NMR (CD₂Cl₂): 144,7 ppm.
¹H-NMR (CD₂Cl₂): 87 (m, 4 H); 6,99 (m, 4 H); 7,08 (m, 2 H); 7,22-7,30 (m, 6 H); 7,30-7,37 (m, 9 H); 7,37-7,45 (m, 8 H) ppm.
¹³C-NMR (CD₂Cl₂): 94,9 (d, *J*_{CP}= 8,1 Hz); 125,0; 127,2; 127,3; 127,4; 127,5; 127,7; 128,6; 128,9; 129,0; 130,5; 130,5; 130,8; 136,6 (d, *J*_{CP}= 4,7 Hz); 138,5; 142,2 (d, *J*_{CP}= 4,6 Hz); 142,3; 147,0 (d, *J*_{CP}= 9,1 Hz) ppm.
ESI-TOF/HRMS: m/e 641,22372 (M+H)⁺.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ∼3 %; *cis*+*trans*-2-Octen: ∼49%; *cis*+*trans*-3-Octen: ∼29%; *cis*+*trans*-Octen-4: ∼16%; gerüstisomere Octene: ∼3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). Der Autoklav wurde bei einem Gesamtgasdruck (Synthesegas: Linde; H₂ (99,999%) : CO (99,997%) = 1:1) von 42 bar für einen Enddruck von 50 bar unter Rühren (1500 U/min) auf die jeweils angegebenen Temperaturen aufgeheizt. Nach Erreichen der Reaktionstemperatur wurde der Synthesegasdruck auf 48,5 bar für einen Enddruck von 50 bar erhöht und das Edukt mit einem in der Druckpipette eingestellten Überdruck von ca. 3 bar zugepresst. Die Reaktion wurde bei konstantem Druck von 50 (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in Tabelle 1 zusammengefasst.

**Tabelle 1:**

| **Ligand** | **p (bar)** | **T (°C)** | **t (h)** | **[Rh] (ppm)** | **L/Rh** | **Ausb. (%)** |
|---|---|---|---|---|---|---|
| **1** | 50 | 120 | 4 | 100 | 5 | 91 |
| **2** | 50 | 120 | 4 | 100 | 5 | 93 |
| **4** | 50 | 120 | 4 | 100 | 5 | 96 |
| **7** | 50 | 120 | 4 | 100 | 5 | 95 |
| **12*** | 50 | 120 | 4 | 100 | 5 | 97 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Mit der erfindungsgemäßen Verbindung konnte eine bessere Ausbeute erzielt werden, als mit den Vergleichsverbindungen **1**, **2**, **4** und **7**.

Anhand der oben beschriebenen Versuche konnte gezeigt werden, dass die gestellte Aufgabe durch die erfindungsgemäßen Verbindungen gelöst wird.

## Patentansprüche

1. Verbindung, welche die allgemeine Struktur **III** aufweist: 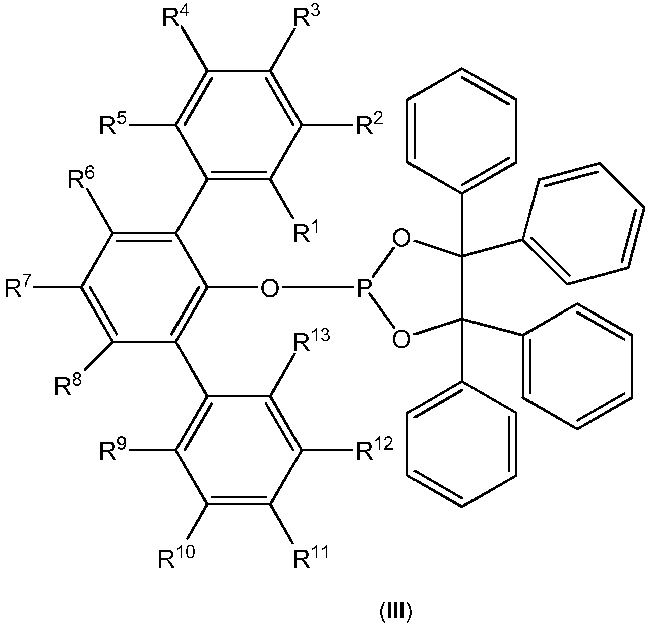 wobei
R¹, R², R³, R⁴, R⁵, R⁶, R⁷, R⁸, R⁹, R¹⁰, R¹¹, R¹², R¹³ ausgewählt sind aus:
- H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.

2. Verbindung nach Anspruch 1,
wobei R¹, R², R³, R⁴, R⁵, R⁶, R⁷, R⁸, R⁹, R¹⁰, R¹¹, R¹², R¹³ ausgewählt sind aus:
-H, -(C₁-C₁₂)-Alkyl, -O-(C₁-C₁₂)-Alkyl, -O-(C₆-C₂₀)-Aryl.

3. Komplex umfassend:
- eine Verbindung nach einem der Ansprüche 1 oder 2,
- ein Metallatom ausgewählt aus: Rh, Ru, Co, Ir.

4. Verwendung einer Verbindung nach einem der Ansprüche 1 oder 2,
zur Katalyse einer Hydroformylierungsreaktion.

5. Verfahren umfassend die Verfahrensschritte:
a) Vorlegen eines Olefins,
b) Zugabe eines Komplexes nach Anspruch 3,
oder einer Verbindung nach einem der Ansprüche 1 oder 2 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure **III**: 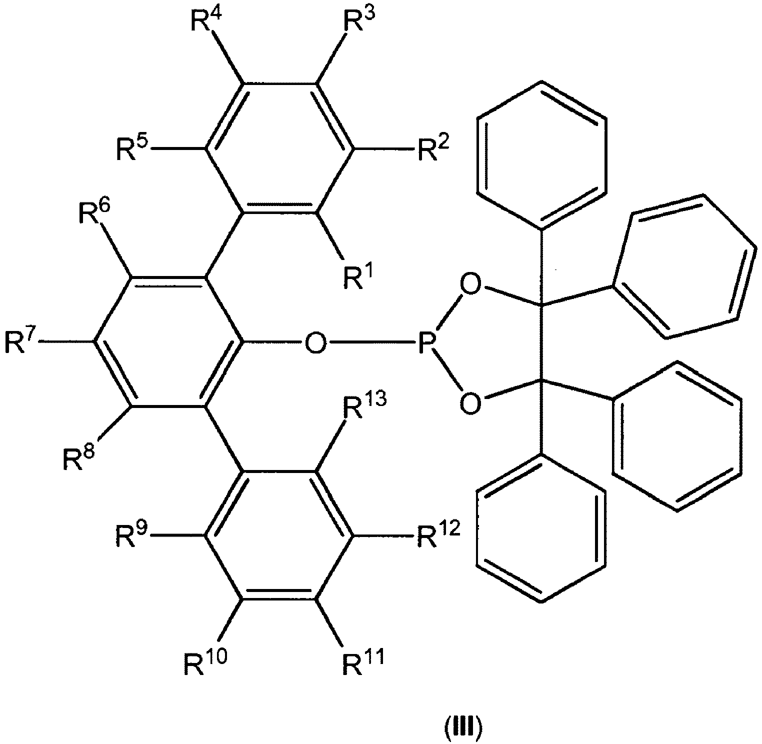 where
R¹, R², R³, R⁴, R⁵, R⁶, R⁷, R⁸, R⁹, R¹⁰, R¹¹, R¹², R¹³ are selected from:
- H, - (C₁-C₁₂)-alkyl, -O- (C₁-C₁₂)-alkyl, -O- (C₆-C₂₀)-aryl, - (C₆-C₂₀)-aryl, -S-alkyl, -S-aryl, halogen, COO- (C₁-C₁₂)-alkyl, CONH- (C₁-C₁₂)-alkyl, -CO- (C₁-C₁₂)-alkyl, -CO- (C₆-C₂₀)-aryl, -COOH, -OH, -SO₃H, -CN, -NH₂, -N[(C₁-C₁₂)-alkyl]₂;
where the alkyl and aryl groups mentioned may be substituted as follows:
substituted - (C₁-C₁₂) -alkyl groups and substituted -(C₁-C₁₂)-alkoxy groups may have one or more substituents depending on their chain length; the substituents are mutually independently selected from - (C₃-C₁₂) - cycloalkyl, - (C₃-C₁₂)-heterocycloalkyl, - (C₆-C₂₀)-aryl, fluorine, chlorine, cyano, formyl, acyl or alkoxycarbonyl,
substituted - (C₆-C₂₀)-aryl groups and - (C₆-C₂₀)-aryl- (C₆-C₂₀)-aryl groups may have one or more substituents depending on their ring size; these substituents are mutually independently selected from -H, -(C₁-C₁₂)-alkyl, -O-(C₁-C₁₂)-alkyl, -O-(C₆-C₂₀)-aryl, -(C₆-C₂₀)-aryl, -halogen, -COO-(C₁-C₁₂)-alkyl, -CONH-(C₁-C₁₂)-alkyl, -(C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H; -SO₃Na,-NO₂, -CN, -NH₂, -N[(C₁-C₁₂)-alkyl]₂.

2. Compound according to Claim 1,
where R¹, R², R³, R⁴, R⁵, R⁶, R⁷, R⁸, R⁹, R¹⁰, R¹¹, R¹², R¹³ are selected from:
- H, - (C₁-C₁₂)-alkyl, -O- (C₁-C₁₂)-alkyl, -O- (C₆-C₂₀)-aryl.

3. Complex comprising:
- a compound according to either of Claims 1 and 2,
- a metal atom selected from: Rh, Ru, Co, Ir.

4. Use of a compound according to either of Claims 1 and 2,
for catalyzing a hydroformylation reaction.

5. Process comprising the following process steps:
a) initially charging an olefin,
b) adding a complex according to Claim 3,
or a compound according to either of Claims 1 and 2 and a substance having a metal atom selected from: Rh, Ru, Co, Ir.
c) feeding in H₂ and CO,
d) heating the reaction mixture, with conversion of the olefin to an aldehyde.

## Revendications

1. Composé, qui présente la structure générale III : 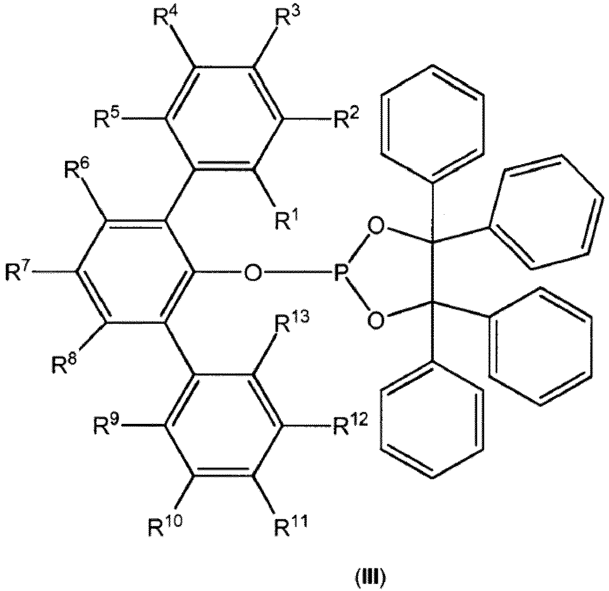 dans laquelle
R¹, R², R³, R⁴, R⁵, R⁶, R⁷, R⁸, R⁹, R¹⁰, R¹¹, R¹², R¹³ sont choisis parmi :
H, alkyle en (C₁-C₁₂), -O-alkyle en (C₁-C₁₂), -O-aryle en (C₆-C₂₀), aryle en (C₆-C₂₀), S-alkyle, S-aryle, halogène, COO-alkyle en (C₁-C₁₂), CONH-alkyle en (C₁-C₁₂), -CO-alkyle en (C₁-C₁₂), -CO-aryle en (C₆-C₂₀), -COOH, -OH, -SO₃H, -CN, -NH₂, -N [alkyle en (C₁-C₁₂)]₂ ;
les groupes alkyle et aryle mentionnés pouvant être substitués de la manière suivante :
les groupes alkyle en (C₁-C₁₂) substitués et les groupes alcoxy en (C₁-C₁₂) substitués peuvent comprendre, en fonction de leur longueur de chaîne, un ou plusieurs substituants ; les substituants étant choisis indépendamment les uns des autres parmi cycloalkyle en (C₃-C₁₂), hétérocycloalkyle en (C₃-C₁₂), aryle en (C₆-C₂₀), fluor, chlore, cyano, formyle, acyle ou alcoxycarbonyle ; les groupes aryle en (C₆-C₂₀) et les groupes aryle en (C₆-C₂₀) -aryle en (C₆-C₂₀) substitués peuvent comprendre, en fonction de la taille du cycle, un ou plusieurs substituants ; ces substituants étant choisis indépendamment les uns des autres parmi H, alkyle en (C₁-C₁₂), -O-alkyle en (C₁-C₁₂), -O-aryle en (C₆-C₂₀), aryle en (C₆-C₂₀), halogène, -COO-alkyle en (C₁-C₁₂), -CONH-alkyle en (C₁-C₁₂), aryle en (C₆-C₂₀)-CON [alkyle en (C₁-C₁₂)]₂,-CO-alkyle en (C₁-C₁₂), -CO-aryle en (C₆-C₂₀), -COOH, -OH, -SO₃H ; -SO₃Na, -NO₂, -CN, -NH₂, -N[alkyle en (C₁-C₁₂)]₂.

2. Composé selon la revendication 1, dans lequel R¹, R², R³, R⁴, R⁵, R⁶, R⁷, R⁸, R⁹, R¹⁰, R¹¹, R¹², R¹³ sont choisis parmi :
H, alkyle en (C₁-C₁₂), -O-alkyle en (C₁-C₁₂), -O-aryle en (C₆-C₂₀).

3. Complexe comprenant :
- un composé selon l'une quelconque des revendications 1 ou 2,
- un atome métallique choisi parmi : Rh, Ru, Co, Ir.

4. Utilisation d'un composé selon l'une quelconque des revendications 1 ou 2 pour la catalyse d'une réaction d'hydroformylation.

5. Procédé comprenant les étapes de procédé suivantes :
a) le chargement d'une oléfine,
b) l'ajout d'un complexe selon la revendication 3,
ou d'un composé selon l'une quelconque des revendications 1 ou 2 et d'une substance qui comprend un atome métallique choisi parmi : Rh, Ru, Co, Ir,
c) l'introduction d'H₂ et de CO,
d) le chauffage du mélange réactionnel, l'oléfine étant transformée en un aldéhyde.
